# EUROPÄISCHE PATENTANMELDUNG

(11) **EP 2 650 401 A1**
(43) Veröffentlichungstag der Anmeldung: **16.10.2013**
(21) Anmeldenummer: 12163588.2
(22) Anmeldetag: 10.04.2012
(51) Int. Cl.: C25B 1/04, C01B 3/00, C07C 1/12, C10L 3/08, C01B 3/08

(54) **Kraftwerk basiertes Methanisierungssystem**

(71) Anmelder: Siemens Aktiengesellschaft, 80333 München (DE)
(72) Erfinder: Hanebuth, Marc, 22297 Hamburg (DE); Lenk, Uwe, 08064 Zwickau (DE); Vortmeyer, Nicolas, 91054 Erlangen (DE)

(57) **Zusammenfassung**

Die Erfindung betrifft ein Kraftwerk basiertes Methanisierungssystem (1), welches neben einem fossil befeuerten Kraftwerk (2) eine Elektrolysiereinheit (3) sowie einen Methanisierungsreaktor (4) aufweist, wobei das Kraftwerk (2) und die Elektrolysiereinheit (3) dazu ausgebildet sind, den Methanisierungsreaktor (4) mit Ausgangsstoffen für eine Methanisierungsreaktion zu versorgen, und wobei die Elektrolysiereinheit (3) sowohl in einem Lade- als auch in einem Entladezustand betrieben werden kann, in welchem Ladezustand die Elektrolysiereinheit (3) mit elektrischem Strom versorgt und gleichzeitig ein chemischer Energiespeicher aufgeladen wird, und in welchem Entladezustand der chemische Energiespeicher entladen wird.

## Beschreibung

Die vorliegende Erfindung betrifft ein Kraftwerk basiertes Methanisierungssystem, welches neben einem fossil befeuerten Kraftwerk eine Elektrolysiereinheit sowie einen Methanisierungsreaktor aufweist. Ferner betrifft die Erfindung Verfahren zum Betrieb eines solchen Methanisierungssystems.

Trotz eines wachsenden Anteils von Stromerzeugungsverfahren basierend auf regenerativen Energiequellen an der Gesamtstromerzeugung bedarf die Deckung der Grundlast in dem öffentlichen Stromversorgungsnetzwerk weiterhin des Betriebs von fossil befeuerten Kraftwerken. Diese setzen insbesondere die in Kohle, Erdgas, Erdöl, oder anderen Rohstoffen enthaltenen chemischen Energien in thermische Energie um, um damit einen Stromerzeugungsprozess zu unterhalten.

Fossil befeuerte Kraftwerke werden aus technischen sowie wirtschaftlichen Gründen bevorzugt kontinuierlich unter Volllast betrieben, so dass sie sich besonders zur Deckung der Grundlast in den öffentlichen Stromversorgungsnetzwerken eignen. Zusätzlich werden den öffentlichen Stromversorgungsnetzwerken aus mit regenerativen Energiequellen gespeisten Stromerzeugungsverfahren in unregelmäßigen Abständen Energie zugeführt. Dies führt mitunter zu starken Schwankungen in der Bereitstellung von elektrischer Energie in den öffentlichen Stromversorgungsnetzwerken. Um diese Schwankungen auszugleichen, ist es erforderlich, zu Zeiten von starker Stromnachfrage zusätzliche elektrische Energie in das Stromversorgungsnetzwerk einzuspeisen, sowie zu Zeiten von herrschendem Überangebot an elektrischer Energie diese aus den öffentlichen Stromversorgungsnetzwerken zu entnehmen.

Um das zeitweise vorherrschende Überangebot von elektrischer Energie in wirtschaftlicher Weise zu nutzen, kann die überschüssige Energie in elektrischer wie auch in chemischer Form zwischengespeichert werden. So ist es bspw. möglich, elektrische Überschussenergie zur synthetischen Herstellung von Methan (substitute natural gas, SNG) zu nutzen. Dieses synthetisch hergestellte Methan lässt sich leicht über aus dem Stand der Technik bekannte katalytische Verfahren aus wenigen Ausgangsstoffen herstellen. Bspw. lässt sich Methan bei geeigneter Wahl der Reaktionsbedingungen aus den Ausgangsstoffen CO und Wasserstoff bzw. CO₂ und Wasserstoff (Sabatier-Prozess) herstellen. Um diese Herstellung jedoch wirtschaftlich zu betreiben, ist es erforderlich, die Synthesereaktion möglichst kontinuierlich sowie unter Volllast vorzunehmen.

Der vorliegenden Erfindung liegt folglich die Aufgabe zugrunde, die Nachteile aus dem Stand der Technik zu vermeiden. Insbesondere liegt der Erfindung die Aufgabe zugrunde, eine im Wesentlichen kontinuierliche Herstellung von synthetischem Methan zu ermöglichen. Ferner soll die synthetische Methanherstellung zu Zeiten eines Überangebotes an elektrischer Energie in den öffentlichen Stromversorgungsnetzwerken wie auch zu Zeiten einer erhöhten Nachfrage von elektrischer Energie in denselben ermöglicht werden. Es soll also eine im Wesentlichen kontinuierliche Methanproduktion ermöglicht werden, die wenigstens teilweise unabhängig von dem elektrischen Stromangebot in den öffentlichen Stromversorgungsnetzwerken ist. Hierbei soll die Energie in erster Linie durch den zeitweise angebotenen Überschussstrom in den öffentlichen Stromversorgungsnetzwerken bereit gestellt werden.

Diese Aufgabe wird durch ein Kraftwerk basiertes Methanisierungssystem gemäß Patentanspruch 1 sowie zwei Verfahren zum Betrieb eines solchen Methanisierungssystems gemäß Patentanspruch 11 und Patentanspruch 12 gelöst.

Insbesondere wird diese Aufgabe durch ein Kraftwerk basiertes Methanisierungssystem gelöst, welches neben einem fossil befeuerten Kraftwerk eine Elektrolysiereinheit sowie einen Methanisierungsreaktor aufweist, wobei das Kraftwerk und die Elektrolysiereinheit dazu ausgebildet sind, den Methanisierungsreaktor mit Ausgangsstoffen für eine Methanisierungsreaktion zu versorgen, und wobei die Elektrolysiereinheit sowohl in einem Lade- als auch in einem Entladezustand betrieben werden kann, in welchem Ladezustand die Elektrolysiereinheit mit elektrischem Strom versorgt und gleichzeitig ein chemischer Energiespeicher aufgeladen wird, und in welchem Entladezustand der chemische Energiespeicher entladen wird.

Weiter wird die Erfindungsaufgabe durch ein Verfahren zum Betrieb eines Methanisierungssystems, insbesondere eines vorab beschriebenen Methanisierungssystems gelöst, welches einem fossil befeuerten Kraftwerk eine Elektrolysiereinheit sowie einen Methanisierungsreaktor aufweist, wobei das Kraftwerk und die Elektrolysiereinheit dazu ausgebildet sind, den Methanisierungsreaktor mit Ausgangsstoffen für eine Methanisierungsreaktion zu versorgen, wobei der Elektrolysiereinheit in einem ersten Schritt Wasser zugeleitet wird, das in einem zweiten Schritt elektrolytisch oder chemisch in Wasserstoff umgewandelt wird und wobei in einem dritten Schritt der Wasserstoff zusammen mit CO₂ aus dem Kraftwerk vermischt wird und das Gemisch von Wasserstoff und CO₂ dem Methanisierungsreaktor als Ausgangsstoff zugeführt wird.

Weiter wird die Erfindungsaufgabe durch ein Verfahren zum Betrieb eines Methanisierungssystems, insbesondere eines vorab beschriebenen Methanisierungssystems gelöst, welches neben einem fossil befeuerten Kraftwerk eine Elektrolysiereinheit sowie einen Methanisierungsreaktor aufweist, wobei das Kraftwerk und die Elektrolysiereinheit dazu ausgebildet sind, den Methanisierungsreaktor mit Ausgangsstoffen für eine Methanisierungsreaktion zu versorgen, wobei der Elektrolysiereinheit in einem ersten Schritt ein Gemisch aus Wasser und CO₂ aus dem Kraftwerk zugeleitet wird, das in einem zweiten Schritt in der Elektrolysiereinheit elektrolytisch oder chemisch entsprechend in Wasserstoff und CO₂ umgewandelt wird, und wobei in einem dritten Schritt das Gemisch von Wasserstoff zusammen mit dem CO₂ dem Methanisierungsreaktor als Ausgangsstoffe zugeführt wird.

Erfindungsgemäß weist also das Kraftwerk basierte Methanisierungssystem neben einem Methanisierungsreaktor zur Herstellung von synthetischem Methan ein fossil befeuertes Kraftwerk auf, welches neben der Bereitstellung von elektrischer Leistung ebenfalls Ausgangsstoffe für die Methanisierungsreaktion zur Verfügung stellen kann. Solche Ausgangsstoffe sind insbesondere CO und CO₂, die aufgrund der Verbrennungsreaktion in dem fossil befeuerten Kraftwerk entstehen.

Weiterhin weist das erfindungsgemäße Methanisierungssystem eine Elektrolysiereinheit auf, die sowohl in einem Lade- als auch in einem Entladezustand betrieben werden kann. In dem Ladezustand wird die Elektrolysiereinheit mit elektrischem Strom entweder aus dem fossil befeuerten Kraftwerk oder aber bevorzugt aus den öffentlichen Stromversorgungsnetzwerken bei Angebot von Überschussstrom versorgt. Gleichzeitig wird ein chemischer Energiespeicher aufgeladen, der während des Entladezustands wieder entladen wird, und damit die für eine Methanisierung benötigte Herstellung des entsprechenden Ausgangsstoffs energetisch versorgt. Laderzustand und Entladezustand können zeitlich unmittelbar aneinander anschließen.

Die Elektrolysiereinheit kann folglich sowohl unter Aufnahme von elektrischem Strom im Ladezustand wie auch unter Abgabe von chemischer Energie im Entladezustand betrieben werden. Damit wird die Bereitstellung von synthetisch hergestelltem Methan zu Zeiten von einem Überangebot an elektrischem Strom in den öffentlichen Stromversorgungsnetzwerken wie auch bei erhöhter Stromnachfrage aus diesen in einem im Wesentlichen kontinuierlichen Betrieb ermöglicht. Der kontinuierliche Betrieb wiederum lässt die Zwischenspeicherung der Ausgangsstoffe überflüssig werden, wodurch die Herstellung bei hoher Auslastung der Anlage sehr wirtschaftlich erfolgen kann.

Erfindungsgemäß ist vorgesehen, dass die Elektrolysiereinheit in dem Ladezustand wenigstens einen Teil der zugeführten elektrischen Energie als chemische Energie in einem chemischen Energiespeicher bevorratet. Demgemäß erfolgt die chemische Speicherung der während des Ladezustands aufgenommenen elektrischen Energie nicht nur indirekt in Form des synthetisch hergestellten Methans in dem Methanisierungsreaktor, sondern die chemische Speicherung erfolgt zudem in der Elektrolysiereinheit selbst. Bei Einstellung von geeigneten Reaktionsbedingungen kann diese chemische Energie wieder in geeigneter Form nutzbar gemacht werden, und erlaubt damit die Energetisierung nachfolgender Prozesse. Insbesondere gewährleistet die Bereitstellung der zwischengespeicherten chemischen Energie den Betrieb der Methanisierungseinheit während des Entladezustands der Elektrolysiereinheit.

Erfindungsgemäß wird die synthetische Methanherstellung mit Ausgangsstoffen aus dem fossil befeuerten Kraftwerk direkt bzw. indirekt sowie mit Ausgangsstoffen aus der Elektrolysiereinheit versorgt. Da sowohl das fossil befeuerte Kraftwerk wie auch die Elektrolysiereinheit im Wesentlichen kontinuierlich diese Ausgangsstoffe bereitstellen können, kann die Methanisierungsreaktion in dem Methanisierungsreaktor ebenfalls im Wesentlichen kontinuierlich verlaufen. Folglich kann im Wesentlichen unabhängig von dem Stromangebot bzw. der Stromnachfrage in den öffentlichen Stromversorgungsnetzwerken synthetisches Methan hergestellt werden, ohne ausschließlich auf die Versorgung mit elektrischer Leistung aus dem fossil befeuerten Kraftwerk eingeschränkt zu sein.

Entsprechend einer besonders bevorzugten Form des Verfahrens wird die Elektrolysiereinheit während des Ladezustands lediglich mit Überschussstrom aus den öffentlichen Stromversorgungsnetzwerken versorgt. Dementsprechend könnte auf eine Versorgung der Elektrolysiereinheit mit elektrischer Leistung aus dem fossil befeuerten Kraftwerk vollständig verzichtet werden. Da jedoch das Stromangebot in den öffentlichen Stromversorgungsnetzwerken während eines Tages mitunter starken Schwankungen ausgesetzt sein kann, kann es ausführungsgemäß auch erforderlich sein, elektrische Leistung aus dem fossil befeuerten Kraftwerk während des Ladezustands der Elektrolysiereinheit abfragen zu müssen.

Im Sinne der vorliegenden Erfindung soll der Begriff des fossil befeuerten Kraftwerks in seiner breitesten Bedeutungsform verstanden werden. Insbesondere sind unter fossil befeuerte Kraftwerke auch Verbrennungsanlagen zur Müllverwertung zu zählen.

Gemäß einer ersten bevorzugten Ausführungsform der Erfindung kann die Elektrolysiereinheit sowohl in dem Lade- als auch in dem Entladezustand wenigstens einen Ausgangsstoff durch Elektrolyse erzeugen. Folglich eignet sich die Elektrolysiereinheit zur im Wesentlichen kontinuierlichen Bereitstellung von Ausgangsstoffen für die Methanisierungsreaktion. Insbesondere ist die Elektrolysiereinheit entsprechend einer Weiterbildung in der Lage, während des Lade- als auch während des Entladezustandes molekularen Wasserstoff zu erzeugen. Dieser kann in dem Methanisierungsreaktor bei geeigneten Reaktionsbedingungen mit dem CO₂ oder CO, bzw. einem Gemisch dieser beiden Stoffe, welche als Verbrennungsprodukte in dem Kraftwerksprozess entstehen, zu synthetischem Methan umgesetzt werden.

Gemäß einer weiteren Ausführungsform des Methanisierungssystems benötigt die Elektrolysiereinheit eine Versorgung mit einem Luftstrom zum Betrieb einer Gaselektrode bzw. zur Abführung des während des Ladezustands gebildeten Sauerstoffs. Insbesondere weist die Elektrolysiereinheit einen Anschluss auf, über welchen der Luftstrom in die Elektrolysiereinheit eingeführt werden kann. Zudem ist der Luftstrom besonders geeignet Wärme aus der Elektrolysiereinheit abzuführen. Ausführungsgemäß kann der Luftstrom auch als allgemeiner Gasstrom ausgeführt sein.

Ausführungsgemäß kann die Elektrolysiereinheit in ihrer Ausgestaltung der in der WO 2011/070006 A1 beschriebenen Batterie entsprechen. Diese Veröffentlichungsschrift sei hiermit ausdrücklich durch Bezugnahme in die vorliegende Anmeldung mit einbezogen. Die dort beschriebene Batterie, welche der vorliegenden Elektrolyseeinheit in ihrem Aufbau im Wesentlichen entspricht, weist zahlreiche Gaskanäle auf, mittels welcher sauerstoffhaltiges Prozessgas an eine Kathode geführt wird. Das sauerstoffhaltige Prozessgas, ist, um das Gefahrenpotential zu verringern wie auch um die Wirtschaftlichkeit zu erhöhen, Luftsauerstoff.

Der in dem Prozessgas enthaltene Sauerstoff wird während des Entladezustands reduziert und tritt durch die ionenleitfähige Kathode hindurch. Aufgrund der vorherrschenden Oxidationspotentiale wandert der reduzierte Sauerstoff weiter durch einen Festkörperelektrolyten sowie zu einer Anode, an welcher der ionische Sauerstoff seine Ladung abgibt und sich zusammen mit molekularem Wasserstoff zu Wasser verbindet. Der Festkörperelektrolyt ist hierbei in vorteilhafterweise zur anionischen Leitfähigkeit geeignet, verhindert jedoch eine elektrische Leitung von Ladungsträgern. Der Festkörperelektrolyt umfasst bspw. ein Metalloxid, wie etwa Zirkonoxid und/oder Ceroxid, welches wiederum mit einem Metall, bspw. Scandium dotiert ist. Aufgrund der Dotierung werden in dem Metalloxid Sauerstoff-Leerstellen erzeugt, die den anionischen Transport von reduziertem Sauerstoff (d.h. zweifach negativ geladenen Sauerstoffatomen) erlauben bzw. die Stabilität des Elektrolythen erhöht.

Aufgrund des an der Anode vorliegenden Reduktionsmittels, bspw. molekularer Wasserstoff, wird der anionische Sauerstoff gemäß der folgenden Gleichung zu H₂O umgesetzt:

H₂ + O²⁻ → H₂O + 2 e⁻ (Gl. 1)

Die hierbei frei werdenden Elektronen können an der Anode abgegriffen und einem elektrischen Verbrauchskreis zugeführt werden.

Anders als bei dem vorab dargestellten Entladezustand der in der WO 2011/070006 A1 beschriebenen Batterie erfolgt die Entladung der vorliegenden Elektrolysiereinheit nicht unter Abgabe von elektrischer Energie. Vielmehr wird in der Elektrolysiereinheit im Entladezustand die in dem chemischen Energiespeicher bevorratete chemische Energie dazu genutzt, die Herstellung eines für die Methanisierung benötigten Ausgangsstoffs voran zu treiben.

Zur Bereitstellung von einem Ausgangsstoff für die Methanisierungsreaktion wird hierzu beispielsweise gasförmiges Wasser in einen Tragkörper eingeleitet, der oxidierbares Material, bevorzugt in Form eines elementaren Metalls, aufweist. Das Material kann als Pulver oder auch als poröser Presskörper vorliegen. Bei Reaktion des gasförmigen Wassers mit dem oxidierbaren Material, welches als Reduktionsmittel für das Wasser wirkt, wird elementarer Wasserstoff beispielsweise nach der folgenden Gleichung erzeugt:

Me + H₂O → MeO + H₂ (Gl. 2)

Hierbei steht Me für ein oxidierbares Material, insbesondere ein Metal, und stellt den chemischen Energiespeicher der Elektrolysiereinheit dar. Dieser kann während des Ladezustands durch geeignete Reduktion des oxidierten Materials erzeugt bzw. wieder regeneriert werden. Aufgrund geeignet gewählter Elektronegativitäten des oxidierbaren Materials ist die Neigung des gasförmigen Wassers stärker mit dem Material in dem Tragkörper zu reagieren als etwa mit einem Metall des Anodenmaterials. Dadurch ist das Anodenmaterial vor Korrosion vorteilhaft geschützt. Weitere Details hinsichtlich des konkreten Aufbaus der Batterie bzw. der Elektrolysiereinheit können aus der oben bezeichneten Veröffentlichungsschrift entnommen werden.

Gemäß einer weiteren vorteilhaften Ausführungsform umfasst die Elektrolysiereinheit ein Metall und/oder ein Metalloxid als chemischen Energiespeicher, welche während des Entladezustands oxidiert werden können. Das Metall und/oder das Metalloxid erlauben also die in ihnen gespeicherte chemische Energie während des Entladezustands wieder in geeigneter Weise freizusetzen. Das Metall und/oder Metalloxid kann bevorzugt aus der Gruppe von Lithium, Mangan, Eisen, Titan oder Wolfram sein. Vorzugsweise liegt das Metall und/oder das Metalloxid als Pulver bzw. poröser Presskörper vor. Insbesondere ermöglicht ein solches Metall und/oder Metalloxid eine geeignete Reaktion mit gasförmigem Wasser, wie oben in Gleichung 2 angedeutet. Dadurch wird molekularer Wasserstoff freigesetzt, welcher als Ausgangsstoff für die Methanisierungsreaktion dienen kann.

Gemäß einer weiteren, besonders bevorzugten Ausführungsform des erfindungsgemäßen Methanisierungssystems umfasst die Elektrolysiereinheit ein Metalloxid, welches während des Ladezustands reduziert werden kann. Nach erfolgter Reduzierung liegt das Metalloxid in einer relativ niedrigeren Oxidationsstufe vor, bzw. in reiner metallischer Form, und kann damit einen chemischen Energiespeicher zur Verfügung stellen, welcher bei Entladung die chemische Energie für die chemische Umwandlung in der Elektrolysiereinheit liefert. Durch die Reduzierung des Metalloxids wird ferner Sauerstoff freigesetzt, der seinerseits wieder als Oxidationsmittel Verwendung finden kann. Entsprechend der weiter oben beschriebenen Batterie, die in ihrem Aufbau der vorliegenden Elektrolysiereinheit entsprechen kann, kann der von dem Metalloxid umfasste Sauerstoff zur Bildung von molekularem Sauerstoff geeignet sein, wobei der molekulare Sauerstoff gewissermaßen als Nebenprodukt während des Ladezustands abgegeben wird. Während des Ladezustands wird somit das Metalloxid in das elementare Metall bzw. in ein verhältnismäßig niederwertigeres Metalloxid reduziert, welches wiederum während des Entladezustands als chemischer Energiespeicher bereitstehen kann.

Entsprechend einer weiteren bevorzugten Ausführungsform des Methanisierungssystems weist die Elektrolysiereinheit eine Zuleitung auf, die dazu ausgebildet ist, die Elektrolysiereinheit mit Wasser, insbesondere mit Wasserdampf zu versorgen. Das Wasser bzw. der Wasserdampf sind als Prozessstoff bzw. Prozessgas vorgesehen, die erlauben, die Elektrolysiereinheit in einem Lade- bzw. Entladezustand zu betreiben. Bevorzugt kann Wasserdampf auch während des Entladezustands als Transportstoff für elementaren Sauerstoff vorgesehen sein. Durch Reaktion mit bspw. einem Metall zu einem Metalloxid wird der Sauerstoff chemisch gebunden und gewährleistet folglich die chemische Speicherung von elektrischer Energie. Ebenso kann das Wasser bzw. der Wasserdampf zur Bereitstellung von elementarem Sauerstoff während des Ladezustandes dienen, wobei durch Abgabe von Sauerstoff der für die Methanisierungsreaktion erforderliche Wasserstoff bereitgestellt werden kann. Wasser bzw. Wasserdampf sind hierbei kostengünstig und in Bezug auf ihre Handhabung verhältnismäßig ungefährlich.

Gemäß einer weiteren Ausführungsform der Erfindung umfasst die Elektrolysiereinheit einen Festkörperelektrolyten, welcher insbesondere zwei elektrische Elektroden elektrisch isolierend voneinander trennt, jedoch eine vorbestimmte Ionenleitfähigkeit, insbesondere eine Anionenleitfähigkeit aufweist. Der Festkörperelektrolyt gewährleistet in vorteilhafter Weise eine elektrische Isolierung, welche die Voraussetzung für einen kontrollierten elektrischen Betrieb der Elektrolysiereinheit darstellt. Aufgrund der selektiven Ionenleitfähigkeit kann ein kontrollierter Entladezustand wie auch Ladezustand erreicht werden. Gleichzeitig verhindert der Festkörperelektrolyt die Vermischung von Prozessgasen, die bspw. während des Lade- bzw. Entladezustandes mit einer der beiden elektrischen Elektroden und/oder beiden elektrischen Elektroden wechselwirken.

Gemäß einer weiteren Ausführungsform des erfindungsgemäßen Methanisierungssystems ist die Elektrolysiereinheit für einen Betrieb für wenigstens 500 °C, insbesondere von wenigstens 600 °C und bevorzugt zwischen 600 °C und 800 °C geeignet. Die hohen Betriebstemperaturen gewährleisten einen effizienten Lade- bzw. Entladebetrieb und folglich eine effiziente Bereitstellung von Ausgangsstoffen für die Methanisierungsreaktion. Weiterhin kann vorteilhaft die Abwärme der Elektrolysiereinheit als Abwärme etwa zur Vorwärmung der Ausgangsstoffe dienen, bevor diese in den Methanisierungsreaktor eingeführt werden.

Entsprechend einer besonders bevorzugten Ausführungsform des erfindungsgemäßen Methanisierungssystems weist das Kraftwerk eine CO₂-Abtrennvorrichtung auf, welche dazu ausgebildet ist, CO₂ aus einem Abgasstrom des Kraftwerks abzutrennen und gasförmiges CO₂ als Ausgangsstoff für die Methanisierungsreaktion im Methanisierungsreaktor und/oder für die Elektrolyse für die Elektrolysiereinheit bereitzustellen. Die CO₂-Abtrennvorrichtung gewährleistet folglich die insbesondere selektive Aufbereitung des Abgasstroms des Kraftwerks um den Ausgangsstoff bereitstellen zu können, welcher während der Methanisierungsreaktion zu synthetischem Methan umgesetzt wird. Andere verunreinigende Stoffe werden hierbei in der CO₂-Abtrennvorrichtung nicht gezielt zur Nutzung abgetrennt, und tragen folglich in dem Methanisierungsreaktor nicht oder nicht Wesentlich zu Verunreinigungen bei. Die selektive Abtrennung von CO₂ erhöht den Wirkungsgrad, mit welchem synthetisches Methan hergestellt werden kann. Zudem erhöht sie die Reinheit des in dem Methanisierungsreaktor hergestellten synthetischen Methans. Weiterhin ermöglicht die gezielte Abtrennung von CO₂ aus dem Abgasstrom des Kraftwerks eine im Wesentlichen mengenkontrollierte Zuführung von CO₂ in den Methanisierungsreaktor.

Gemäß einer weiteren bevorzugten Ausführungsform des Methanisierungssystems ist weiterhin eine Wärmebrücke vorgesehen, welche dazu ausgebildet ist, thermische Energie vom Methanisierungsreaktor zur Elektrolysiereinheit zu leiten. Die Wärmebrücke ist insbesondere dafür vorgesehen, positive wie negative thermische Energie zu leiten, d.h. die Wärmeleitung kann in beide Richtungen erfolgen. Da die Methanisierungsreaktion typischerweise stark exotherm ist, kann die dabei frei werdende Wärme zur Vorwärmung der Elektrolysiereinheit eingesetzt werden. Diese Wärme wird der Elektrolysiereinheit mittels der Wärmebrücke zugeleitet. Ebenso eignet sich die bei der Methanisierungsreaktion frei werdende Wärme zur Konditionierung des in die Elektrolysiereinheit eingeleiteten Wassers, um dieses bspw. zu verdampfen. Ausführungsgemäß kann also die während der Methanisierungsreaktion anfallende Wärme zum Teil bzw. größtenteils zum Betrieb der Elektrolysiereinheit genutzt werden. Ebenso ist es denkbar, dass die Wärme zum Vorheizen von Prozessgasströmen genutzt wird, welche der Elektrolysiereinheit zugeleitet werden. Diese Wärmenutzung erhöht folglich den Gesamtwirkungsgrad des ausführungsgemäßen Methanisierungssystems. Zudem vereinfacht es das Wärmemanagement bei Betrieb des Methanisierungssystems.

Gemäß einer weiteren Ausführungsform des erfindungsgemäßen Methanisierungssystems ist eine Wasserrückführung vorgesehen, welche dazu ausgebildet ist, Wasser, welches nach erfolgter Methanisierungsreaktion angefallen ist, wieder zur Elektrolysiereinheit bzw. zur Zuleitung, die wenigstens dazu ausgebildet ist, die Elektrolysiereinheit mit Wasser zu versorgen, zurückzuführen. Die Wasserrückführung vermindert damit einerseits einen übermäßigen Wasserverbrauch, sowie andererseits einen unerwünschten Energieverbrauch zur thermischen Konditionierung des der Elektrolysiereinheit zugeführten Wassers. Da das nach der Methanisierungsreaktion angefallene Wasser typischerweise noch einen großen Wärmeinhalt aufweist, erfordert es relativ wenig Energie dieses Wasser für den Einsatz in der Elektrolysiereinheit erneut zu verdampfen. Eine Wasserrückführung ermöglicht somit ein effizientes Stoff- und Wärmemanagement.

Gemäß einer ersten bevorzugten Ausführungsform des erfindungsgemäßen Verfahrens ist vorgesehen, die Elektrolysiereinheit, insbesondere kontinuierlich in entweder einem Lade- oder in einem Entladezustand zu betreiben. Der kontinuierliche Betrieb der Elektrolysiereinheit gewährleistet die kontinuierliche Bereitstellung von Ausgangsstoffen für die Methanisierungsreaktion. Folglich ist es nicht erforderlich, diese Ausgangstoffe vor Zuführung in dem Methanisierungsreaktor zu bevorraten. Der kontinuierliche Betrieb der Elektrolysiereinheit ermöglicht zudem eine hohe wirtschaftliche Effizienz des Betriebs.

Gemäß einer Weiterführung dieses Verfahrens kann vorgesehen sein, dass der Lade- und der Entladezustand sich abwechseln. Durch ein solches Abwechseln von Lade- und Entladezustand kann ein effizienter kontinuierlicher Betrieb erreicht werden. Hierbei wird die Elektrolysiereinheit insbesondere dann von dem Entladezustand in einen Ladezustand überführt, wenn während des energieautarken Entladezustandes nicht mehr ausreichende Mengen an Ausgangsstoffen für die Methanisierungsreaktion bereitgestellt werden können. Folglich kann durch eine gezielte Alternierung von Entladezustand und Ladezustand eine vorteilhaft hohe Effizienz bei der Ausführung des Gesamtverfahrens erreicht werden.

Gemäß einer weiteren besonders bevorzugten Ausführungsform des erfindungsgemäßen Verfahrens werden die Ausgangsstoffe im Wesentlichen in stöchiometrischen Mengen als Gemisch dem Methanisierungsreaktor zugeführt. Bevorzugt sind die Abweichungen von den erforderlichen stöchiometrischen Mengen geringer als 20 %, besonders bevorzugt weniger als 10 % und ganz besonders bevorzugt weniger als 5 %. Dem Methanisierungsreaktor werden also im Wesentlichen nur solche Mengen an Ausgangsstoffen zugeführt, die auch während der Methanisierungsreaktion vollständig umgesetzt werden können. Folglich ist das in dem Methanisierungsreaktor erzeugte synthetische Methan zu einem relativ geringeren Grad von Fremdstoffen verunreinigt, so dass eine aufwendige Gastrennung nach Herstellung des synthetischen Methans nicht erforderlich ist. Ist die Umsetzung im Methanisierungsreaktor vollständig, so wird das aus dem Methanisierungsreaktor entnommene synthetische Methan lediglich durch Wasser verunreinigt sein. Das Wasser, welches bei dem im Methanisierungsreaktor vorherrschenden Temperaturen typischerweise dampfförmig vorliegt, kann jedoch leicht auskondensiert werden und anschließend bspw. über eine geeignete Wasserrückführung der Elektrolysiereinheit erneut zugeleitet werden.

Gemäß einer weiteren bevorzugten Ausführungsform des erfindungsgemäßen Verfahrens erfolgt keine Zwischenspeicherung der Ausgangsstoffe nach Entnahme dieser aus der Elektrolysiereinheit. Ausführungsgemäß liegt also ein Flusssystem vor, welches auf die Bereitstellung von Zwischenspeichern verzichten kann. Ein solches Flusssystem ist insbesondere dann vorteilhaft zu verwirklichen, wenn sowohl Elektrolysiereinheit als auch der Methanisierungsreaktor kontinuierlich fließend betrieben werden können. Dies gewährleistet wiederum eine hohe Verfahrenseffizienz.

Gemäß einer weiteren Ausführungsform des erfindungsgemäßen Verfahrens, werden die Produkte, welche aus dem Methanisierungsreaktor entnommen werden, nicht einem Verfahren zur Rückverstromung zugeführt. Ebenso ist es möglich, die Produkte einer Infrastruktur für die Handhabung von Erdgas als synthetisches Gas direkt zuzuführen. Eine Rückverstromung, die thermische und elektrische Verlustleistung zur Folge haben würde, kann folglich vermieden werden.

Gemäß einer weiteren Verfahrensmodifikation kann vorgesehen sein, dass der Methanisierungsreaktor kontinuierlich betrieben wird. Der kontinuierliche Betrieb gewährleistet insbesondere eine hohe Verfahrenseffizienz sowie eine gewünschte kontinuierliche Bereitstellung von synthetischem Methan.

Nachfolgend soll die vorliegende Erfindung anhand von Ausführungsbeispielen erläutert werden. Hierbei sei darauf hingewiesen, dass die Konkretisierung der Erfindung in den Ausführungsbeispielen keine Einschränkung hinsichtlich des allgemein beanspruchten Gegenstandes darstellt. Ferner werden die in den Ausführungsbeispielen erläuterten Merkmale einzeln für sich wie auch in Verbindung mit anderen Merkmalen beansprucht.

Weiterhin sei darauf hingewiesen, dass die nachfolgenden Figuren lediglich schematische Darstellungen sind. Dies stellt jedoch keine Einschränkung hinsichtlich der konkreten Ausführbarkeit der vorliegenden Erfindung dar.

Hierbei zeigen die Figuren Folgendes:
Fig. 1 eine schematische Darstellung des erfindungsgemäßen Methanisierungssystems gemäß einer ersten Ausführungsform;
Fig. 2 eine schematische Darstellung des erfindungsgemäßen Methanisierungssystems gemäß einer zweiten Ausführungsform;
Fig. 3 eine schematische Darstellung einzelner chemischer Reaktionen und Abläufe während des Betriebs der Elektrolysiereinheit in einem Ladezustand;
Fig. 4 eine schematische Darstellung einzelner chemischer Reaktionen und Abläufe während des Betriebs der Elektrolysiereinheit in einem Entladezustand;
Fig. 5 ein schematisches Flussdiagramm zur Veranschaulichung einer ersten Ausführungsform des erfindungsgemäßen Verfahrens.
Fig. 6 ein schematisches Flussdiagramm zur Veranschaulichung eines erfindungsgemäßen Verfahren gemäß einer zweiten Ausführungsform;

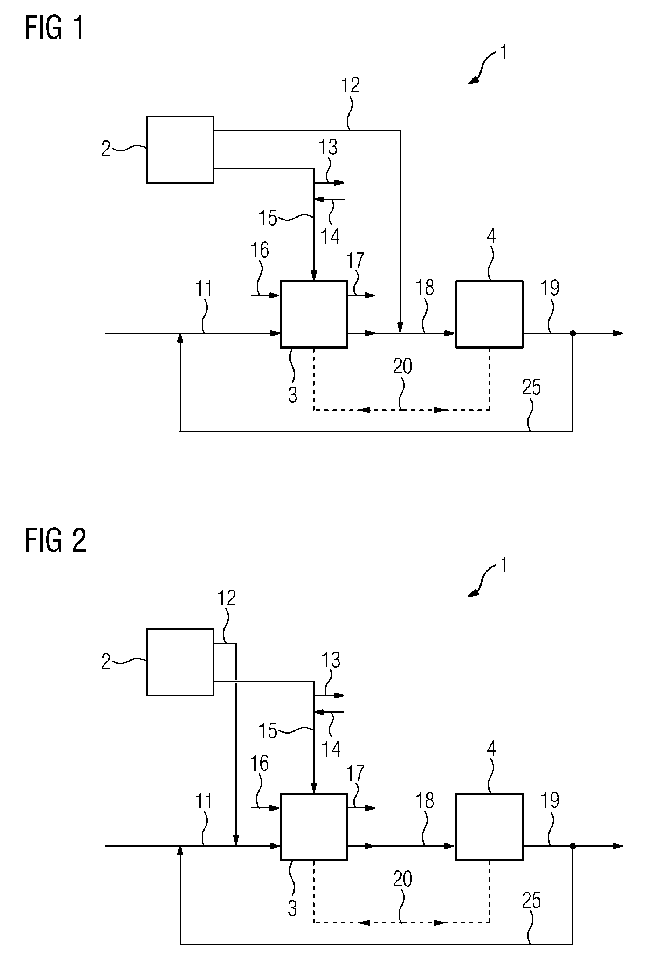

Fig. 1 zeigt eine erste Ausführungsform des erfindungsgemäßen Methanisierungssystems 1, welches neben einem fossil befeuerten Kraftwerk 2 noch eine Elektrolysiereinheit 3 aufweist. Sowohl das Kraftwerk 2 als auch die Elektrolysiereinheit 3 sind dafür vorgesehen, Ausgangsstoffe 10 (vorliegend nicht gezeigt) bereit zu stellen und einem Methanisierungsreaktor 4 zuzuführen, in welchen die Ausgangsstoffe 10 in geeigneter Weise chemisch in synthetisches Methan umgesetzt werden.

Hierbei stellt das fossil befeuerte Kraftwerk 2 in einer Versorgungsleitung 12 gasförmiges CO₂ bereit. Bevorzugt wurde das bereitgestellte CO₂ in dem Kraftwerk 2 mittels einer nicht weiter gezeigten CO₂-Abtrennvorrichtung aus einem Abgasstrom des Kraftwerks 2 abgetrennt. Zur elektrischen Stromversorgung der Elektrolysiereinheit 3 ist ferner eine Stromversorgungsleitung 15 vorgesehen, welche erlaubt, die Elektrolysiereinheit 3 mit Strom aus dem fossil befeuerten Kraftwerk 2 zu versorgen. Alternativ und/oder ergänzend kann auch über eine Netzentnahmeleitung 14 elektrische Leistung aus den öffentlichen Stromversorgungsnetzwerken entnommen werden, und der Elektrolysiereinheit 3 zugeführt werden. Ebenfalls kann aus dem Kraftwerk 2 über eine Netzabgabeleitung 13 den öffentlichen Stromversorgungsnetzwerken elektrische Energie zugeführt werden.

Für den Betrieb der Elektrolysiereinheit 3 in einem Entlade- /Ladezustand kann es erforderlich sein, dieser Luft über eine Luftzufuhrleitung 16 zuzuführen. Das zugeführte Gas wird nach bestimmungsgemäßer Benutzung der Elektrolysiereinheit 3 bzw. während der bestimmungsgemäßen Benutzung der Elektrolysiereinheit 3 wieder aus der Luftableitung 17 abgeführt. Nach der Entnahme kann dieses Gas beispielsweise der Umgebungsluft wieder zugeführt werden. Es dient einerseits einer geeigneten Wärmeabführung und andererseits dazu, den während des Ladezustands gebildeten Sauerstoff abzuführen.

Weiter erfordert der Betrieb der Elektrolysiereinheit 3 die Zuführung eines geeigneten Prozessstoffes, welcher über die Zuleitung 11 der Elektrolysiereinheit 3 zugeleitet werden kann. Ausführungsgemäß ist dieser Prozessstoffes Wasser bzw. Wasserdampf, welcher in der Elektrolysiereinheit 3 während des Entladezustands und des Ladezustands wenigstens zum Teil in Wasserstoff umgesetzt wird. Der durch Elektrolyse oder chemische Umsetzung hergestellte Ausgangsstoff 10 für die Methanisierungsreaktion wird mittels der Übertragungsleitung 18 dem Methanisierungsreaktor 4 zugeleitet. Wird in der Elektrolysiereinheit 3 keine vollständige Umsetzung von Wasser erreicht, so werden die nicht umgesetzten Mengen wenigstens teilweise auch mit in den Methanisierungsreaktor 4 zusammen mit dem elektrolytisch hergestellten Wasserstoff befördert. Gemäß der vorliegenden Ausführungsform mündet die Versorgungsleitung 12 zur Bereitstellung von CO₂ in die Übertragungsleitung 18, so dass sich darin der Wasserstoff zusammen mit dem CO₂ vermischen kann. Dieses Gemisch wird anschließend dem Methanisierungsreaktor 4 zugeleitet, in welchem die Ausgangsstoffe 10 zu synthetischem Methan umgesetzt werden. Das so hergestellte synthetische Methan wird mittels einer Produktabteilung 19 aus dem Methanisierungsreaktor abgeleitet.

Während der Methanisierungsreaktion, die stark exotherm abläuft, wird Wärme frei, welche über die Wärmebrücke 20 an die Elektrolysiereinheit 3 in geeigneter Weise geleitet werden kann. Die so bereitgestellte Wärme kann in der Elektrolysiereinheit 3 zur Vorwärmung der eingeführten Prozessgase bzw. zur Vorwärmung des Wassers bzw. zur Verdampfung des Wassers dienen. Aufgrund dieser thermischen Konditionierung herrscht in der Elektrolysiereinheit 3 ein im Vergleich zur Umgebung deutlich höheres Temperaturniveau vor. Die Temperaturerhöhung hat wiederum eine Verminderung der Elektrolysespannung zur Folge, was seinerseits einen verbesserten Wirkungsquerschnitt bei der Bereitstellung der Ausgangsstoffe 10 zur Folge hat.

Das aus der Produktableitung 19 entnommene Produkt, idealerweise ein Gemisch aus synthetischem Methan und Wasser, erfordert eine geeignete Abtrennung von Wasser aus dem Produktstrom. Dies kann beispielsweise durch eine vorteilhafte Auskondensierung des in dem Produktstrom enthaltenen Wassers erreicht werden, wobei das Wasser über eine Wasserrückführung 25 wieder der Zuleitung 11 zur Bereitstellung an die Elektrolysiereinheit 3 zugeleitet werden kann.

Fig. 2 zeigt eine weitere Ausführungsform des erfindungsgemäßen Methanisierungssystems 1. Es unterscheidet sich von dem in Fig. 1 gezeigten Methanisierungssystem 1 lediglich dahingehend, dass das durch die Versorgungsleitung 12 bereitgestellte CO₂ nicht in die Übertragungsleitung 18 zur Zuführung an den Methanisierungsreaktor 4 vorgesehen ist, sondern zur Einführung in die Zuleitung 11, um der Elektrolysiereinheit 3 zugeführt zu werden. Folglich wird über die Zuleitung 11 der Elektrolysiereinheit 3 ein Gemisch aus CO₂ sowie Wasser als Prozessstoffe zugeleitet, wobei beide Stoffe in der Elektrolysiereinheit 3 durch Elektrolyse entsprechend umgesetzt werden können. Wird CO₂ in der Elektrolysiereinheit 3 zu CO umgesetzt, so wird Wasser, gemäß den obigen Ausführungen, in Wasserstoff umgesetzt. Beide Stoffe, Wasserstoff und CO, werden als Ausgangsstoffe 10 aus der Elektrolysiereinheit 3 über die Übertragungsleitung 18 dem Methanisierungsreaktor 4 zu geleitet. In dem Methanisierungsreaktor 4 werden beide Stoffe als Ausgangsstoffe entsprechend in synthetisches Methan umgesetzt. Wird in der Elektrolysiereinheit 3 keine vollständige Umsetzung von Wasser und CO2 erreicht, so werden die nicht umgesetzten Mengen auch mit in den Methanisierungsreaktor 4 zusammen mit dem Wasserstoff und dem CO befördert.

Hierbei können in der Ausführungsform gemäß Fig. 2, wie auch in der Ausführungsform gemäß Fig. 1 über die Versorgungsleitung 12 stöchiometrische Mengen an CO₂ zugegeben werden. D.h. über die Versorgungsleitung 12 wird gerade so viel CO₂ hinzugegeben, dass die dem Methanisierungsreaktor 4 zugeführte Ausgangsstoffe 10 stöchiometrisch, d.h. im Wesentlichen vollständig, umgesetzt werden können. Um die Mengen an CO₂ geeignet einstellen zu können, können vorliegend nicht weiter gezeigte Einstellmittel, insbesondere Ventile, vorgesehen sein.

Ferner veranschaulicht die Ausführungsform gemäß Fig. 1 und gemäß Fig. 2, dass keine Zwischenspeicher vorgesehen sind, in welchen die Ausgangsstoffe 10 vor Zuführung in den Methanisierungsreaktor zwischengespeichert werden müssten. Vielmehr befinden sich sowohl das fossil befeuerte Kraftwerk 2 als auch die Elektrolysiereinheit 3 wie auch der Methanisierungsreaktor 4 in im Wesentlichen kontinuierlichem Betrieb, so dass aus der Produktableitung 19 kontinuierlich synthetisch hergestelltes Methan entnommen werden kann.

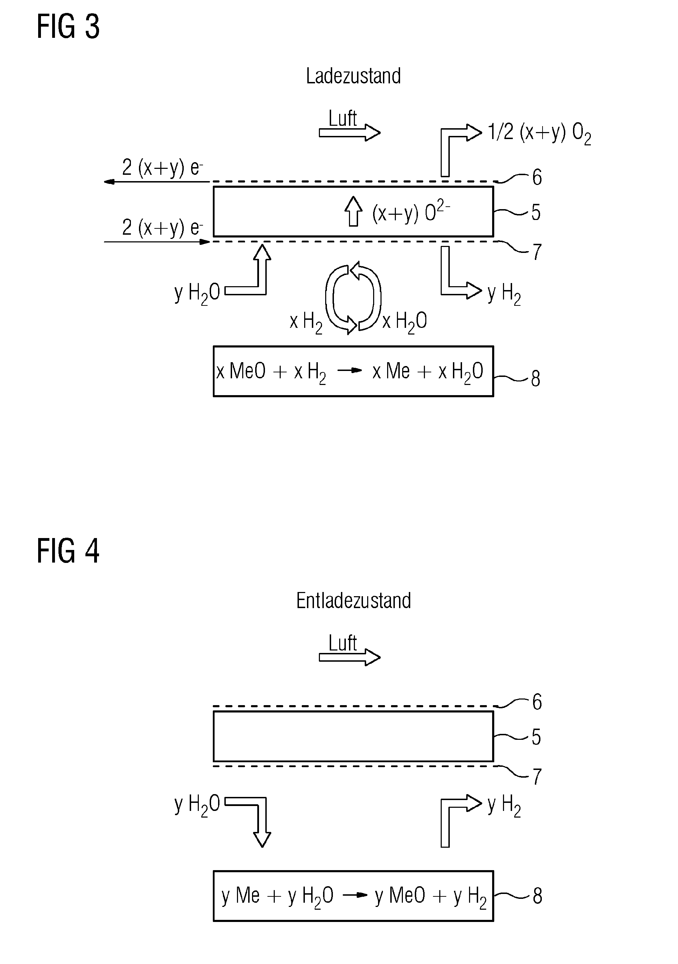

Die in der Elektrolysiereinheit 3 während eines Ladezustands ablaufenden chemischen Reaktionen und Vorgänge sind in Fig. 3 schematisch veranschaulicht. Hierbei umfasst die Elektrolysiereinheit 3 eine Anordnung aus einer ersten elektrischen Elektrode 6 sowie einer zweiten elektrischen Elektrode 7, welche beide elektrisch isolierend voneinander durch einen Festkörperelektrolyten 5 getrennt sind. Die erste elektrische Elektrode 6 ist hierbei mit Luft als Prozessgas in direktem Kontakt. Die erste elektrische Elektrode kann beispielsweise eine Substanz mit Perowskitstruktur umfassen. Sie kann eine Schichtdicke zwischen 10 und 200 µm aufweisen, wobei sie bevorzugt etwa 50 µm aufweist. Der Festkörperelektrolyt 5 ist typischerweise als metalldotiertes Metalloxid ausgeführt und weist eine Schichtdicke von typischerweise zwischen 20 und 100 µm, bevorzugt 50 µm auf. Die zweite Elektrode 7 kann als Metall-Keramik-Verbundmaterial, einem sogenannten Cermet ausgestaltet sein, wobei vorteilhafte Metalle Lithium, Mangan, Eisen, Titan, Wolfram oder Nickel sein können.

Ausführungsgemäß ist die zweite Elektrode 7 mit gasförmigem Wasser in Kontakt. In demselben Reaktionsraum, befindet sich ebenfalls ein Metalloxid (MeO), welches durch molekularen Wasserstoff in elementares Metall (Me) und Wasser umgesetzt werden kann. Das Metall dient hierbei als chemischer Energiespeicher 8 während des in Fig. 4 gezeigten Entladezustands. Während des vorliegend gezeigten Ladezustandes wird jedoch Metalloxid wieder in die zur chemischen Speicherung geeignete Form, nämlich das Metall, reduziert.

Während des Landezustands liegt zwischen der ersten Elektrode sowie der zweiten Elektrode 7 ein elektrisches Potenzial an, das an der zweiten Elektrode 7 für einen Überschuss an Elektronen (e⁻) sorgt. Durch Aufnahme zweier Elektronen aus der zweiten Elektrode 7 wird Wasser zu Wasserstoff (H₂) reduziert, wobei gleichzeitig ein zweifach negativ geladenes Sauerstoffanion entsteht. Dieses Anion wandert von der zweiten Elektrode 7 durch den Festkörperelektrolyten 5 zur ersten Elektrode 6, wo es erneut seine elektrische Ladung abgibt und zu molekularem Sauerstoff reagiert. Der molekulare Sauerstoff wird zusammen mit der Luft, welche mit der ersten Elektrode in Kontakt ist, abgeführt.

Der aus der an der zweiten Elektrode 7 durch Zersetzung entstehende Wasserstoff reagiert seinerseits mit dem Metalloxid, wobei Metall in elementarer Form, sowie Wasser entsteht. Das in dieser Reaktion entstehende Wasser kann seinerseits wiederum an der zweiten Elektrode 7 zu Wasserstoff reduziert werden, wobei erneut ein Sauerstoffanion gebildet wird, welches zur ersten Elektrode 6 durch den Festkörperelektrolyten 5 wandert.

Das aus der Reaktion des Wasserstoffs mit dem Metalloxid frei werdende Wassermolekül wird folglich an der zweiten Elektrode 7 erneut in Wasserstoff umgesetzt. Demnach unterhält sich die Reduktion des Metalloxids zu elementarem Metall selbst, indem nämlich für jedes Wasserstoffmolekül ein Wassermolekül entsteht, welches wiederum erneut an der zweiten Elektrode 7 ein Wasserstoffmolekül entstehen lässt. Anders verhält es sich jedoch auf das von außen eingeleitete Wasser, welches an der zweiten Elektrode 7 zu Wasserstoff umgesetzt wird, ohne jedoch dass dieser erneut zur Reduktion des Metalloxids beiträgt. Ausführungsgemäß wird dieser so freigesetzte Wasserstoff aus der Elektrolysiereinheit 3 als Ausgangsstoff 10 für die Methanisierungsreaktion in dem Methanisierungsreaktor 4 abgeleitet.

Wird die Elektrolysiereinheit 3 nun anstelle eines Ladezustands in einem Entladezustand betrieben, laufen die in Fig. 4 schematisch dargestellten Abläufe ab. Anders als während des Ladezustands liegt während des Entladezustands zwischen der ersten Elektrode 6 sowie der zweiten Elektrode 7 kein elektrisches Potenzial an, welches die Vorgänge antreiben würde. Vielmehr wird ein Anionenfluss durch den Festkörperelektrolyten 5 unterbunden. Eine Unterbindung der Anionenwanderung durch den Festkörperelektrolyten 5 erfolgt beispielsweise durch eine Verhinderung der Entnahme von elektrischem Strom über die beiden Elektroden 6 und 7.

Während des Entladezustands wird Wasser in die Elektrolysiereinheit 3 eingeführt, das mit dem Metall unter Oxidation des Metalls zu Wasserstoff chemisch umgesetzt wird. Da dieser Wasserstoff an der zweiten Elektrode 7 nicht weiter reagiert, kann er als Ausgangsstoff 10 der Methanisierungsreaktion dem Methanisierungsreaktor 4 zugeführt werden. Das Metall übernimmt somit die Rolle des chemischen Energiespeichers 8, welcher die Energie für die ablaufende Elektrolyse von Wasser zu Wasserstoff zur Verfügung stellt.

Gemäß den ablaufenden Reaktionen, welche für den Ladezustand in Fig. 3 und für den Entladezustand in Fig. 4 innerhalb der Elektrolysiereinheit dargestellt sind, wird stets ein Anteil an Wasser in einen Anteil an Wasserstoff umgesetzt. Dieser kann als Ausgangsstoff 10 für die Methanisierungsreaktion im Methanisierungsreaktor 4 dienen. Ausführungsgemäß kann also sowohl während des Ladezustand als auch während es Entladezustands Wasserstoff als Ausgangsstoff bereitgestellt werden, unabhängig von dem Betriebszustand.

Die im Energiespeicher 8 ablaufenden Reaktionen sind vorliegend exemplarisch für ein zweiwertiges Metall dargestellt. Dies soll jedoch keine Einschränkung darstellen. Vielmehr lässt sich das Prinzip auf andere geeignete Substanzen übertragen. Die Reaktionsgleichungen ändern sich dann entsprechend.

Neben Wasser als Ausgangsstoff für die Elektrolyse in der Elektrolysiereinheit 3 kann ebenso ein Gemisch aus CO₂ und Wasser zugeführt werden, wobei CO₂ in CO und Wasser in Wasserstoff umgesetzt wird. Hierbei wird CO₂ durch Abgabe eines Sauerstoffatoms an der zweiten Elektrode 7 in CO umgesetzt. Zudem ist es durchaus denkbar, dass die Umsetzungen in der Elektrolysiereinheit 3 nicht vollständig erfolgen, also die Umsetzungsgrade nicht 100% betragen.

Weiterhin kann es auch sinnvoll sein, einen Anteil des aus Wasser elektrolytisch erzeugten Wasserstoffs der Elektrolysiereinheit 3 erneut zuzuführen, um eine Alterung der Elektroden, insbesondere der im Entladezustand als Kathode betriebenen Elektrode, zu verhindern bzw. diese Alterung umzukehren.

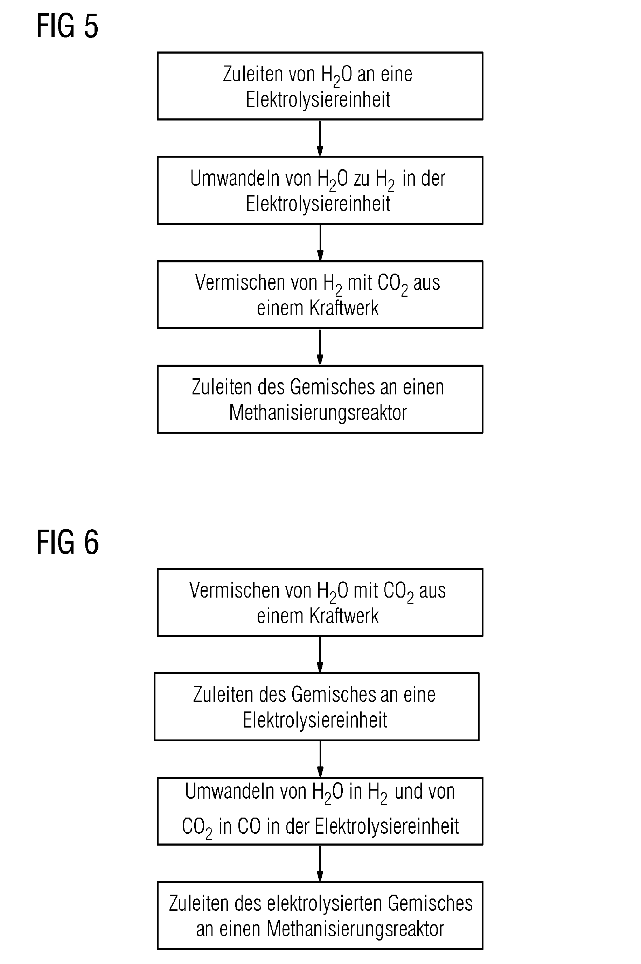

In Fig. 5 ist eine schematische Abfolge von Verfahrensschritten gezeigt, zur Darstellung einer ersten Ausführungsform des erfindungsgemäßen Verfahrens in einem Flussdiagramm. Hierbei wird in einem ersten Verfahrensschritt Wasser an eine Elektrolysiereinheit 3 (vorliegend nicht bezeichnet) geleitet, damit dieses dort in einem zweiten Schritt elektrolytisch oder chemisch in Wasserstoff umgewandelt wird. Diese Umwandlung kann wie unter Fig. 3 und 4 bereits erläutert, sowohl während eines Entladezustands als auch während eines Ladezustands der Elektrolysiereinheit 3 erfolgen. In einem dritten Schritt wird der so erzeugte Wasserstoff mit CO₂ aus einem Kraftwerk 2 (vorliegend nicht bezeichnet) vermischt. Das CO₂ stammt hierbei bevorzugt aus einer CO₂-Abtrennvorrichtung, die in das Kraftwerk 2 integriert bzw. diesem nachgeschaltet ist. Beide Gase, Wasserstoff und CO₂, werden in einem weiteren Schritt als Gemisch dem Methanisierungsreaktor 4 (vorliegend nicht bezeichnet) zugeleitet, um in diesem in einer Methanisierungsreaktion zu synthetischem Methan umgesetzt zu werden.

In Fig. 6 ist eine weitere Ausführungsform des erfindungsgemäßen Verfahrens als Flussdiagramm dargestellt. Hierbei unterscheidet sich das in Fig. 6 dargestellte Verfahren von dem in Fig. 5 dargestellten Verfahren lediglich dahingehend, dass der Elektrolysiereinheit in einem zweiten Schritt ein Gemisch aus Wasser und CO₂ zugeleitet wird. Die Elektrolysiereinheit 3 wandelt in einem dritten Schritt elektrolytisch oder chemisch Wasser in Wasserstoff und CO₂ in CO um. Das so erhaltene Gemisch wird in einem vierten Schritt einem Methanisierungsreaktor 4 zugeleitet, so dass die darin enthaltenen Ausgangsstoffe 10 in einer Methanisierungsreaktion zu synthetischem Methan umgesetzt werden können.

## Patentansprüche

1. Kraftwerk basiertes Methanisierungssystem (1), welches neben einem fossil befeuerten Kraftwerk (2) eine Elektrolysiereinheit (3) sowie einen Methanisierungsreaktor (4) aufweist, wobei das Kraftwerk (2) und die Elektrolysiereinheit (3) dazu ausgebildet sind, den Methanisierungsreaktor (4) mit Ausgangsstoffen (10) für eine Methanisierungsreaktion zu versorgen, und wobei die Elektrolysiereinheit (3) sowohl in einem Lade- als auch in einem Entladezustand betrieben werden kann, in welchem Ladezustand die Elektrolysiereinheit (3) mit elektrischem Strom versorgt und gleichzeitig ein chemischer Energiespeicher (8) aufgeladen wird, und in welchem Entladezustand der chemische Energiespeicher (8) entladen wird.

2. Methanisierungssystem gemäß Anspruch 1,
**dadurch gekennzeichnet, dass** die Elektrolysiereinheit (3) sowohl in dem Lade- als auch in dem Entladezustand wenigstens einen Ausgangsstoff (10) durch Elektrolyse erzeugen kann.

3. Methanisierungssystem gemäß einem der vorher gehenden Ansprüche,
**dadurch gekennzeichnet, dass** die Elektrolysiereinheit (3) ein Metall und/oder ein Metalloxid als chemischen Energiespeicher (8) umfasst, welche während des Entladezustands oxidiert werden können.

4. Methanisierungssystem gemäß einem der vorher gehenden Ansprüche,
**dadurch gekennzeichnet, dass** die Elektrolysiereinheit (3) ein Metalloxid umfasst, welches während des Ladezustands reduziert werden kann.

5. Methanisierungssystem gemäß einem der vorher gehenden Ansprüche,
**dadurch gekennzeichnet, dass** die Elektrolysiereinheit (3) eine Zuleitung (11) aufweist, die dazu ausgebildet ist, die Elektrolysiereinheit (3) mit Wasser, insbesondere mit Wasserdampf zu versorgen.

6. Methanisierungssystem gemäß einem der vorher gehenden Ansprüche,
**dadurch gekennzeichnet, dass** die Elektrolysiereinheit (3) einen Festkörperelektrolyten (5) umfasst, welcher insbesondere zwei elektrische Elektroden (6,7) elektrisch isolierend voneinander trennt, jedoch eine vorbestimmte Ionenleitfähigkeit, insbesondere eine Anionenleitfähigkeit aufweist.

7. Methanisierungssystem gemäß einem der vorher gehenden Ansprüche,
**dadurch gekennzeichnet, dass** die Elektrolysiereinheit (3) für einen Betrieb bei wenigstens 500°C, insbesondere von wenigstens 600°C und bevorzugt zwischen 600°C und 800°C geeignet ist.

8. Methanisierungssystem gemäß einem der vorher gehenden Ansprüche,
**dadurch gekennzeichnet, dass** das Kraftwerk (2) eine CO₂-Abtrennvorrichtung aufweist, welche dazu ausgebildet ist, CO₂ aus einem Abgasstrom des Kraftwerks (2) abzutrennen und gasförmiges CO₂ als Ausgangsstoff (10) für die Methanisierungsreaktion im Methanisierungsreaktor (4) und/oder für die Elektrolyse in der Elektrolysiereinheit (3) bereit zu stellen.

9. Methanisierungssystem gemäß einem der vorher gehenden Ansprüche,
**dadurch gekennzeichnet, dass** weiterhin eine Wärmebrücke (20) vorgesehen ist, welche dazu ausgebildet ist, thermische Energie vom Methanisierungsreaktor (4) zur Elektrolysiereinheit (3) zu leiten.

10. Methanisierungssystem gemäß einem der vorher gehenden Ansprüche,
**dadurch gekennzeichnet, dass** eine Wasserrückführung (25) vorgesehen ist, welche dazu ausgebildet ist, Wasser, welches nach erfolgter Methanisierungsreaktion angefallen ist, wieder zur Elektrolysiereinheit (3) bzw. zur Zuleitung (11), die wenigstens dazu ausgebildet ist, die Elektrolysiereinheit (3) mit Wasser zu versorgen, zurück zu führen.

11. Verfahren zum Betrieb eines Methanisierungssystems (1), insbesondere eines in den vorher gehenden Ansprüchen beschriebenen Methanisierungssystems (1), welches neben einem fossil befeuerten Kraftwerk (2) eine Elektrolysiereinheit (3) sowie einen Methanisierungsreaktor (4) aufweist, wobei das Kraftwerk (2) und die Elektrolysiereinheit (3) dazu ausgebildet sind, den Methanisierungsreaktor (4) mit Ausgangsstoffen (10) für eine Methanisierungsreaktion zu versorgen,
wobei der Elektrolysiereinheit (3) in einem ersten Schritt Wasser zugeleitet wird, das in einem zweiten Schritt elektrolytisch oder chemisch in Wasserstoff umgewandelt wird, und wobei in einem dritten Schritt der Wasserstoff zusammen mit CO2 aus dem Kraftwerk (2) vermischt wird und das Gemisch von Wasserstoff und CO2 dem Methanisierungsreaktor (4) als Ausgangsstoffe (10) zugeführt wird.

12. Verfahren zum Betrieb eines Methanisierungssystems (1), insbesondere eines in den vorher gehenden Ansprüchen beschriebenen Methanisierungssystems (1), welches neben einem fossil befeuerten Kraftwerk (2) eine Elektrolysiereinheit (3) sowie einen Methanisierungsreaktor (4) aufweist, wobei das Kraftwerk (2) und die Elektrolysiereinheit (3) dazu ausgebildet sind, den Methanisierungsreaktor (4) mit Ausgangsstoffen (10) für eine Methanisierungsreaktion zu versorgen,
wobei der Elektrolysiereinheit (3) in einem ersten Schritt ein Gemisch aus Wasser und CO2 aus dem Kraftwerk (2) zugeleitet wird, das in einem zweiten Schritt in der Elektrolysiereinheit (3) elektrolytisch oder chemisch entsprechend in Wasserstoff und CO umgewandelt wird, und wobei in einem dritten Schritt das Gemisch von Wasserstoff zusammen mit dem CO dem Methanisierungsreaktor (4) als Ausgangsstoffe (10) zugeführt wird.

13. Verfahren gemäß einem der vorher gehenden Ansprüche 11 oder 12,
**dadurch gekennzeichnet, dass** die Elektrolysiereinheit (3) kontinuierlich, insbesondere kontinuierlich in entweder einem Lade- oder in einem Entladezustand betrieben wird.

14. Verfahren gemäß einem der Ansprüche 11 bis 13, **dadurch gekennzeichnet, dass** die Ausgangsstoffe (10) im Wesentlichen in stöchiometrischen Mengen als Gemisch dem Methanisierungsreaktor (4) zugeführt werden.

15. Verfahren gemäß einem der Ansprüche 11 bis 14, **dadurch gekennzeichnet, dass** der Methanisierungsreaktor (4) kontinuierlich betrieben wird.
